# EUROPEAN PATENT APPLICATION

(11) **EP 4 212 149 A1**
(43) Date of publication of application: **19.07.2023**
(21) Application number: 22151974.7
(22) Date of filing: 18.01.2022
(51) Int. Cl.: A61K 9/20, A61K 9/28

(54) **ORAL DOSAGE FORMS OF ACID-LABILE SALT AND METHODS AND USES RELATED THERETO**

(71) Applicant: RIJKSUNIVERSITEIT GRONINGEN, 9712 CP Groningen (NL); Academisch Ziekenhuis Groningen, 9713 GZ Groningen (NL); UMC Utrecht Holding B.V., 3584 CS Utrecht (NL)
(72) Inventor: FRIJLINK, Henderik Willem, 9713 AV Groningen (NL); EISSENS, Anko Cornelus, 9713 AV Groningen (NL); VAN GOOR, Harm, 9713 GZ Groningen (NL); MULDER, Douwe Johannes, 9713 GZ Groningen (NL); JOLES, Jacob Alexander, 3584 CX Utrecht (NL)
(74) Representative: V.O.

(57) **Abstract**

The invention relates to the field of oral drug delivery and more in particular, to oral dosage forms for delivery of a high dose of a therapeutically active acid-labile salt, such as sodium thiosulfate (STS). Provided is a pharmaceutical oral dosage form for controlled delivery of an acid-labile salt, the dosage form comprising a core comprising the acid-labile salt in a polymer matrix comprising a combination of (i) a pH-sensitive hydrophilic methacrylic acid-methyl methacrylate copolymer and (ii) a carbomer, wherein the acid-labile salt makes up at least 50wt% of the core; and wherein the outer surface of the core is provided with an enteric coating layer.

## Description

The invention relates to the field of oral drug delivery and more in particular, it relates to oral dosage forms for delivery of a high dose of a therapeutically active acid-labile salt, such as sodium thiosulfate.

Sodium thiosulfate (Na₂S₂O₃; STS) is an intermediate oxidation product of H₂S, produces H₂S through a non-enzymatic and an enzymatic glutathione-dependent pathway. Glutathione disulfide (GSSG), H₂S and labeled sulfite are produced when mitochondria are incubated with oxygen, glutathione (GSH), and [35S]thiosulfate, respectively [14]. In humans, the use of STS has been approved for treatment of calciphylaxis and in the treatment of cyanide poisoning and cisplatin toxicity [15]. V asodilating and anti-oxidative properties of STS have also been described [16]. These beneficial effects of STS make it an attractive therapeutic candidate for reducing the damaging effects of hypertension, obesity and chronic kidney disease (CKD), all key risk factors for heart failure with preserved ejection fraction (HFpEF).

In recent experimental studies, STS reduced blood pressure, proteinuria, kidney and heart damage as a single treatment regimen in hypertensive rats [17,18]. More recently, we confirmed that oral STS as a single treatment regimen has renoprotective effects in hypertensive rats. These effects were amplified when STS was used as an add on to oral renin angiotension system (RAS) inhibition (Lisinopril) [19]. Cardioprotective effects such as improved systolic function, left ventricular hypertrophy, cardiac fibrosis and oxidative stress were also observed in the same model [20].

These data show that the endogenous compound thiosulfate, derived from exogenously administered STS, has protective properties and improves end-organ damage in cardiovascular and renal disease. The attractive aspect of STS is that it can be safely administered to humans. It has been in use in large intravenous doses for decades in patients with cyanide poisoning and in patients with calciphylaxis.

Besides our recent studies in hypertensive rats [19, 20], various other studies have shown that STS can also be safely administered orally. Oral administration of STS as maintenance therapy for calciphylaxis was well tolerated with no reported side effects [21]. Oral STS has also been used to reduce calcium phosphate nephrolithiasis in hypercalciuric rats [22] and we recently found antihypertensive effects in pregnant Dahl salt-sensitive rats, a model of pre-eclampsia [23].

Oral drug delivery is considered as an often-preferred route of (chronic) administration for medicines as compared to parenteral routes, since it is convenient, relatively low cost, and patient compliance is high. Intravenous administration is not feasible for chronic use. The vast majority of pharmaceutical products are being used orally. Moreover, the oral route is suitable for using controlled release technology since it allows for the use of many different dosage forms and excipients.

Despite these advantages, STS is currently not regularly administered to human subjects via the oral route. This is because the development of safe and efficient oral dosage forms of STS has thus far been hampered by a number of challenges. First, STS suffers from degradation when in contact with the acid content of the stomach, resulting in a low and irregular bioavailability. Second, STS is typically administered in a high dose, in general 300 - 1200 mg or sometimes even more. Once in the intestinal tract, the high dose of STS should not be released at once, because this may cause gastro-intestinal problems such as intestinal cramps or diarrhea. Third, conventional enteric coatings suffer from the risk of an unwanted dose dump of the STS in the stomach, when the coating layer is damaged. Fourth, in view of the fact that STS is a highly soluble salt, formulation of a core-tablet with a slow release profile is difficult. This is especially caused by the high mass (typically at least 300 mg per dosage unit, but 600 or even 900 mg may also be desired) of STS that is needed, which leaves only little room for formulation with co-ingredients/excipients allowing for the desired release rate. A high dose leaves little room for the use of drug release modifying excipients if one wishes to design a product of reasonable size/mass that can conveniently be swallowed by a patient.

The inventors therefore set out to develop an acid-resistant slow-release formulation that allows for oral administration and controlled release of an acid-labile salt, such as STS, in the intestine. In particular, they aimed at providing a release profile that is characterized by an initial lag-time during which no or only a minimal release occurs, which prevents the release of STS in the stomach, followed by a slow release phase, during which the drug is gradually released in the small intestine. Further, the formulation should have an advantageously high content of acid-labile salt.

It was surprisingly found that these goals can be met by incorporating the acid-labile salt in a matrix comprising a combination of distinct pH-sensitive hydrophilic polymers to obtain a tablet core, and providing the core with an outer enteric coating.

Accordingly, in one aspect the invention provides a pharmaceutical oral dosage form for controlled release of an acid-labile salt, the dosage form comprising a core comprising the acid-labile salt in a polymer matrix comprising a combination of (i) a pH-sensitive hydrophilic methacrylic acid-methyl methacrylate copolymer and (ii) a carbomer, wherein the acid-labile salt makes up at least 50wt% of the core; and wherein the outer surface of the core is provided with an enteric coating layer.

An oral dosage form according to the present invention has not been taught or suggested in the prior art. Oral formulations of STS comprising an enteric coating to protect against stomach acid are well known in the art. These mostly involve the coating of regular (gelatin) capsules comprising powdered STS (with optional carriers) with an enteric coating, including Eudragit type polymers, or with a wax. See for example EP2451435, EP2756757, WO2015/056013 and US2013/0136725. However, the art is silent about incorporating a high amount of STS (> 50w% of the dosage unit) in a matrix of distinct hydrophilic pH-sensitive polymers as herein disclosed.

Whereas matrix tablets composed of mixtures of hydrophilic polymers, including "controlled release matrix tablets" on the basis of Carbopol/Eudragit, have been disclosed in the art for (colon-specific delivery) of other types of drugs, it appears that these involve a drug dosage <200 mg per matrix tablet, which is well below the current drug loading of at least 50 w%. Moreover, the drugs are of hydrophobic nature and not, like STS, a water soluble salt.

A number of matrix materials including carbomer have been tested in oral formulations of water soluble (salt) active agents. See for example Senel *et al.* [24] who investigated factors affecting the formulation of sustained release potassium chloride tablets. Formulations containing hydrophilic (methylcellulose, carbomer), plastic (polyvinyl chloride), and wax (glycerol palmitostearate) matrix materials at concentrations of 10, 15 and 20%, incorporated with KCl as active ingredient and insoluble excipients were prepared. Analysis of in vitro release mechanisms indicated that the most suitable results were obtained by methylcellulose and glycerol palmitostearate.

Also, Eudragit RS-PM has been used as matrix forming material in KCl tablets [25]. This polymer is hydrophobic and distinct from the hydrophilic polymer used in the current polymer blend.

### Acid-labile salt

A dosage form of the present invention is, among others, characterized by a relatively high amount of acid-labile salt. One or more salts can be combined. Due to the unique polymer matrix composition combined with an enteric outer coating, the core of an oral dosage form can accommodate for a large proportion (50 wt% or more) of one or more therapeutic salt(s). For example, the salt or combination of salts makes up at least 55 wt%, preferably at least 60 wt%, more preferably at least 65w% of the tablet core, the other core constituents being polymer matrix materials and optional pharmaceutically acceptable excipient(s), like filler, lubricant, plasticizer.

As used herein, the term "acid-labile salt" or "acid sensitive salt" refers to any therapeutically or prophylactically effective salt which is inactivated or degraded when exposed to acid (< pH 5) environment, in particular to gastric fluid. As will be appreciated by a person skilled in the art, the present invention is advantageously used to formulate an oral dosage form of an acid-labile salt which requires a high single dosage, e.g. of at least 300 mg, preferably at least 400 mg, more preferably at least 600 mg. Suitable test conditions for assessing *in vitro* sensitivity of a salt (in unprotected form) to acid conditions are known in the art, and include hydrochloric acid solutions e.g. in the pH range of 1 to 3, and simulated gastric fluid USP containing pepsin and sodium chloride. In one aspect, the acid-labile salt is destroyed to degrees ranging from 30-100% in the first 15-30 minutes after exposure to acid. See for example the test systems described in Boggiano et al. [26], reporting gastric acid inactivation of erythromycin stearate.

In one embodiment, the oral dosage from comprises a thiosulfate salt, which encompasses any naturally occurring or synthetically produced salt of thiosulphate. For example, the invention provides slow release oral compositions comprising sodium thiosulfate (STS) and/or lanthanum thiosulfate. In a preferred aspect, it comprises STS. In one embodiment, it comprises at least 250 mg, preferably at least 300 mg, more preferably at least 400 mg thiosulphate, such as 400, 450, 500, 550, 600 or 650 mg.

In a further embodiment, the oral dosage form comprises a salt of a substituted benzimidazole. Omeprazole, lansoprazole, pantoprazole, rabeprazole and esomeprazole are examples of substituted benzimidazoles, which are inhibitors of the proton pump, an enzyme also called H+/K+-ATPase, found on the surface of acid-secreting parietal cells in the stomach. The substituted benzimidazoles are referred to as proton pump inhibitors or PPI's. Conventionally, omeprazole and lansoprazole are administered as gelatin capsules containing enteric-coated granules of the drug to prevent naturally occurring gastric acid from denaturing these drugs, which are acid-labile.

In a still further embodiment, the salt is an erythromycin slat, such as erythromycin stearate. Erythromycin, a macrolide antibiotic, is used to treat a wide variety of bacterial infections. As shown by Boggiano et al. (supra), this drug is rapidly inactivated by (simulated) gastric acid. For treatment of bacterial infections in adults, a typical dosage regimen is 400 milligrams (mg) every 6 hours or 800 mg every 12 hours. Depending on the severity of the infection, the dose may be increased as needed up to 4000 mg per day. Since typical dosages comprise 400 mg or more erythromycin per tablet, it is highly preferred to formulate erythromycin salt in an oral dosage form of the present invention.

Yet another acid-labile salt that is advantageously incorporated in an oral dosage form as herein disclosed is a salt of methenamine. Methenamine is a heterocyclic organic compound with a cage-like structure similar to adamantane. Methenamine is highly soluble in water and polar solvents, and its molecular constitution is similar to adamantane compounds with tetrahedral cage like structure. In acidic conditions, methenamine decomposes to formaldehyde and ammonia. In one aspect, the invention provides an oral dosage form for the controlled release of methenamine hippurate, which is the hippuric acid salt of methenamine. Other salts include methenamine mandelate and methenamine sulfosalicylate.

In salt form, methenamine is typically used long-term to treat chronic urinary tract infections and to prevent the recurrence of infections. WO2004/110361 discloses the use of (oral) methenamine for the treatment of cancer. It is proposed to administer 2.0 grams of methenamine (urised, 500 mg/tablet, x 4 tablets/dose) orally twice per day. Altinoz *et al.* [27] describe that methenamine may be an effective adjuvant in treatment of systemic cancers and glioblastoma.

### Core matrix

The core of an oral dosage form according to the invention is also characterized by a matrix formed by a unique combination of release controlling hydrophilic polymers. These comprise (i) a pH-sensitive hydrophilic methacrylic acid-methyl methacrylate copolymer and (ii) a carbomer.

As is demonstrated herein below, this polymer matrix ensures a controlled release of the active agent(s) incorporated therein. Furthermore, the interaction of the hydrophilic polymers with the enteric coating results in an unexpected synergistic decrease in the release rate of the salt from the core. The matrix may contain one or more further polymers or other excipients. In a preferred embodiment, the polymers in the matrix consist of a binary mixture of a pH-sensitive methacrylic acid-methyl methacrylate copolymer and a carbomer. pH-sensitive hydrophilic methacrylic acid-methyl methacrylate copolymers for use in the polymer matrix are known in the art. They are typically used to provide effective and stable enteric coatings with a fast dissolution in the upper bowel. Exemplary copolymers include those marketed under the tradename EUDRAGIT^{®} L 100-55 CAS number: 25212 - 88 - 8); Eudragit^{®} S 100, Eudragit^{®} S 12.5, Eudragit^{®} S 12.5 P (Evonik Industries, Essen, Germany), and similar anionic copolymers. In one embodiment, the copolymer is a poly[methacrylic acid, methyl methacrylate] in a 1:1 or 1: 2 polymer ratio.

The second release controlling polymer in the matrix is a carbomer. Carbomers are synthetic high-molecular-weight polyacrylic acids cross-linked with allyl sucrose or allyl pentaerythritol and typically contain between 56 and 68% w/w carboxylic acid groups and 0.75%-2% of cross-linking agents. Depending upon the degree of cross-linking and manufacturing conditions, various grades of carbomers are available. Each grade has its own significance and applicability in pharmaceutical dosage forms. In one aspect, a polymer matrix as herein disclosed comprises a carbomer that has a low residual solvent content.

Preferably, carbomer is selected from the CARBOPOL^{®} series, known acrylic acid polymerisates having high molecular weights, for example, carbomer 941 (CARBOPOL^{®} 71 G, CARBOPOL^{®} 971) and carbomer 934 (CARBOPOL^{®} 974). Carbopol 934 is cross-linked with allyl sucrose and polymerized in benzene. Carbopol 71, 971, and 974 are cross-linked with allylpentaerythritol and polymerized in ethyl acetate. For example, the carbomer is a synthetic high-molecular-weight polyacrylic acid cross-linked with allyl pentaerythritol, which is commercially available as "Carbopol 71G", or a carbomer similar thereto. "Carbopol 71G" appears as a granular solid form of the carbomer.

With the salt representing the largest weight fraction of the tablet core, the polymer matrix typically makes up most of the remaining weight of the tablet core, typically about 20 to 40 wt%. Preferably, the pH-sensitive hydrophilic methacrylic acid-methyl methacrylate copolymer makes up 5-30% of the core-mass and/or the carbomer makes up 5-35% of the core mass.

The relative ratio between the release controlling polymers in the matrix can vary according to needs or circumstances. For example, the release behavior of drugs from matrix tablets typically depends on the size and/or shape of the tablets, leading to different volume to surface ratio's, the (relative) amounts of release controlling polymers may vary to obtain the desired release profile.

In one embodiment, the weight ratio between (i) the pH-sensitive methacrylic acid-methyl methacrylate copolymer and (ii) the carbomer is in the range of 1 : 0.4 to 1: 3. Preferably, it is in the range of 1 : 0.55 to 1: 2.5, more preferably 1 : 0.65 to 1 : 2. In one embodiment, the weight ratio between (i) the pH-sensitive methacrylic acid-methyl methacrylate copolymer and (ii) the carbomer is in the range of 1 : 0.4 to 1: 1, preferably in the range of 1 : 0.55 to 1: 1, more preferably 1 : 0.65 to 1 : 1. In another embodiment, the weight ratio between (i) the pH-sensitive methacrylic acid-methyl methacrylate copolymer and (ii) the carbomer is in the range of 1 : 1 to 1: 3, preferably in the range of 1 : 1 to 1: 2.5, more preferably 1 : 1 to 1 : 2. In addition to the salt(s) and polymer matrix, the core may contain one or more pharmaceutically acceptable excipients, such as a filler, plasticizer, lubricant and/or a glidant, preferably selected from calcium sulfate dihydrate, magnesium oxide, sucrose, microcrystalline cellulose, hydrogenated fatty acids, magnesium stearate, glycerol behenate, precipitated silica, magnesium hydroxide, calcium oxide, polyalcohols, talc, polyethylene glycol, and mixtures thereof. Typically, if present, the excipient(s) in total make up to about 5 wt%, e.g. 0.1-3 wt%, or 1 to 2.5 wt%, of the core.

### Enteric coating

According to the invention, the oral dosage form for controlled delivery of an acid sensitive salt contains an outer enteric coating which serves to protect the core against gastric acids and unwanted premature release. In other words, the enteric coating refers to a barrier applied to oral medication that prevents its dissolution or disintegration in the acid gastric environment. An enteric coating may comprise a layer or layers which are stable upon exposure to acid, including stomach acid, but which degrade or break down in more basic conditions. In order to prevent salt release in the stomach, the enteric coating layer may be designed to release acid-labile salt only at a pH above 5.0 preferably at a pH above 6.0, most preferably at a pH above 6.5.

Suitable enteric coatings may comprise, for example, fatty acids, waxes, shellac, plastics, and/or plant fibers. Enteric coatings for use in this invention may comprise methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers (for example such as, Eudragit L 30 D-55, Eudragit L 100-55, Eudragit S 100, Eastacryl 30d, Kollicoat Mae 30 Dp, Kollicoat Mae 100 P), Sodium alginate and stearic acid. In a preferred embodiment, the enteric coating material comprises a pH-sensitive methacrylic acid-methyl methacrylate copolymer, e.g. Eudragit L 100-55 or a similar copolymer.

Enteric coats may further comprise, for example plasticizers, surfactants, pigments, anti-adherents, opacifying agents, colorants and the like -all of which are routinely used in the preparation of solutions and/or suspensions for use as coatings or enteric coatings. Plasticizers for use in this invention may comprise polyethylene glycol, tributyl sebacate, acetylated monoglycerides, glycerin, triacetin, phthalate esters, castor oil, sorbitol, polysorbates such as sorbitan monolaurate (Span 20), sorbitan monopalmitate, sorbitan monostearate, sorbitan monoisostearate; citrate ester type plasticizers like triethyl citrate, citrate phthalate; propylene glycol, glycerin, polyethylene glycol (low & high molecular weight), dibutyl sebacate, tributyl sebacate; dibutyltartrate, dibutyl phthalate, glycerol palmitosterate and mixtures thereof.

Of specific interest is the use of an enteric coating layer comprising one or more swellable disintegrants. This concept, known in the art as "ColoPulse technology", was previously disclosed in WO2007/013794. The technology offers a pH-controlled release system. It combines a pH-sensitive polymer with a swellable disintegrant, to result in an irreversible disruption of the coating once the pH threshold has been passed. The ColoPulse delivery system allows for a fast and transient release of a substance in response to a given pH change, which typically is governed by the type of pH-sensitive polymer used. Preferred disintegrants include crosslinked sodium carboxymethyl cellulose (AC-DI-SOL^{®}), sodium starch glycolate (EXPLOTAB^{®} PRIMOJEL^{®}) and crosslinked polyvinylpolypyrrolidone (Plasone-XL^{®}). To ensure a pH-controlled release of the substance surrounded by the coating layer, the swellable agent should only become accessible to fluid upon an initial dissolution of the matrix in which it is embedded. Thus, the swellable agent must initially be shielded from fluids by the pH-sensitive coating material to prevent premature swelling and disintegration of the coating layer. In other words, the swellable agent must be present in the coating layer in a non-percolating way. As disclosed in WO2007/013794, this can be achieved when use is made of a suspension of particles of swellable agent in a solution of pH-sensitive coating material, such as enteric coating polymers or other pH-sensitive coating polymers.

Accordingly, in one embodiment the dosage form comprises (a) a pH-sensitive methacrylic acid-methyl methacrylate copolymer, preferably Eudragit L100-55, and (b) a swellable disintegrant. Further excipients may be present as well, such as talc.

### Dissolution Behavior

As is exemplified herein below, a dosage form of the invention has a unique dissolution behavior. More specifically, the enteric coating protects the core against (gastric) acid, and the tablet core ensures a slow release of salt from the polymer matrix at neutral pH. Furthermore, a synergistic interaction between core and coating was found to overcome the intrinsic weakness in the performance of enteric coatings and prevents unwanted dose dump under acid conditions.

The dissolution behavior is readily determined using in vitro testing, for example using a "two stage dissolution test," the first two hours are carried out using a dissolution medium that has a pH of below 5.0. For example, a citrate buffer of pH 2.8 is used. At 2 hours the dissolution medium is changed to a medium that comprises a phosphate buffer and has a pH of about 6.8. This second stage can last 60 to 240 minutes, depending on the dissolution rate of the salt. As is understood in the art, the first medium simulates the stomach conditions while the second medium simulates the intestinal conditions. Using the two-stage in vitro dissolution test as described above, the outer enteric coating layer should exhibit essentially no acid-labile salt release, i.e., less than 10%, typically less than 3%, and generally less than or near the limit of detection, in the first medium (e.g. for the first 2 hours).

Other ingredients can be present in the media, such as enzymes, etc., as is known in the art, e.g., in formulating simulated intestinal fluid (SIF). The two media dissolution test is conducted at 37° C. The stirring speed is typically 50-100 rpm, though the speed can be adjusted if necessary for the dissolution testing to give more useful information for a particular embodiment. The amount of salt released from the coated tablet cores (i.e., dissolved into the dissolution media), can be determined by ordinary methods using routine skill, for example using a conductivity assay.

Provided herein is a dosage form having (as determined in a two stage dissolution test described herein above) a dissolution profile wherein up to 10%, preferably essentially none, of the acid-labile salt is released in the first stage and wherein during the second stage 35-65 w% of the dose is released after 90 minutes (after initiation of the second stage) and more than 75 w% of the dose after 180 minutes. For example, the dissolution profile shows that up to 10%, preferably essentially none, of the acid-labile salt is released in the first stage and that it takes at least 120 minutes, preferably at least 130 minutes, before 80 w% of the total dosage of acid-labile salt is released in a gradual way during the second stage. The percentage of release at a point in time refers to the cumulative release up to that point in time, as per the conventional usage of these terms in the art.

### Method of manufacture

The invention also relates to a method for the manufacture of a dosage form according to the invention. The method comprises the steps of:
(i) providing a dry (powder) mixture comprising acid-labile salt, a pH-sensitive hydrophilic methacrylic acid-methyl methacrylate copolymer, a hydrophilic carbomer and optional pharmaceutically acceptable excipient(s), wherein the acid-labile salt makes up at least 50wt% of the mixture;
(ii) processing the dry mixture into a compacted tablet core;
(iii) providing a coating composition comprising an enteric coating polymer and, optionally, a swellable disintegrant, and applying the coating composition onto the tablet core.

In step (i), the tablet core ingredients of choice, i.e. controlled release matrix polymers and acid-labile salt(s) are dry-mixed in the desired weight ratio, optionally together with one or more further pharmaceutical excipients, such as a diluent, a dry binder, compression aids, preservatives and lubricants. In one embodiment, the excipient(s) is added to a main- or premixture of matrix polymers and pharmaceutically active salt(s), yielding the final mixture.

The lubricant may be any substance that can suitably be incorporated into a tablet formulation for prevention of adhesion between the tablet (to be) formed and the equipment used for its formation, such as an extruder, a mold, a press, and the like. Examples of compounds that are regularly used as lubricant in this respect are fatty substances, such as magnesium stearate, sodium stearylfumarate, glycerylbehenate, glyceryl monostearate, calcium stearate, hydrogenated vegetable oil, polyethylene glycol, talc and zinc stearate.

"Mixing" is understood in the context of the invention as meaning a process for combining substances with the aim of achieving a substantially homogeneous distribution of different substances by the action of mechanical forces. Mixing for the purposes of the invention is suitably performed in conventional mixing devices, such as a shaker mixer. In a Turbula type low speed stirring mixer is used. The total mixing time in step (i) is usually 1 to 30 minutes, preferably 2 minutes to 20 minutes, more preferably 5 minutes to 17 minutes.

In one embodiment, the method comprises mixing sodium thiosulfate, Carbopol and Eudragit in a Turbula mixer at about 80-100 rpm for 10-12 minutes. After the addition of the magnesium stearate, mixing can be continued for another 2-4 minutes.

In step (ii), the mixture obtained from step (i) is processed into a tablet (e.g. by compaction or extrusion) using conventional means and methods. In the context of this invention, compaction is usually understood to mean the conversion of a powder mixture by applying pressure, preferably mechanical pressure, into compacted tablet cores. The tablet cores of the present invention can be of any suitable size and shape, for example round, oval, polygonal, or pillow-shaped. According to the present invention it is preferred that the extended release tablets are and of oval oblong or round, biconvex, shape.

In order to control and maintain the required quality, the obtained matrix tablet cores can be subjected to the following in-process controls: tablet mass, hardness, tablet height and friability. In one embodiment, tablet cores e.g. biconvex round tablets of 9-13 mm having a weight of 200 to 1500 mg, preferably 400-700 mg, are compressed with a compression force of about 10 to 40 kN. The tablet crushing strength is preferably at least 100 N, more preferably at least 150 N, e.g. the range of about 170-210 N.

Step (iii) comprises providing the matrix tablet core with an enteric coating to result in an oral dosage form of the invention. To that end, a coating composition comprising one or more enteric coating polymer(s) in a suitable solvent is provided. For example, enteric polymer is dissolved in a volatile solvent, preferably an alcohol, such as ethanol. One or more additives can be added for example plasticizers, surfactants, pigments, anti-adherents, disintegrants, opacifying agents, colorants and the like -all of which are routinely used in the preparation of solutions and/or suspensions for use as coatings or enteric coatings.

In one embodiment, a coating composition based on the ColoPulse technology (see herein above) is prepared. For example, the coating suspension comprises a solvent or solvent mixture in which the coating material is soluble and in which the swellable disintegrant agent is insoluble and non-swollen. Thus, the insoluble particles of swellable agent in the coating suspension are surrounded by coating material in a dissolved form. The coating suspension can be applied to the substance to be coated, e.g. by spraying. After removal of the solvent or solvent mixture, the resultant coating layer consists of a matrix of coating material in which particles of swellable agent are incorporated. In a preferred embodiment, a coating suspension is prepared using an organic solvent. Most pH-sensitive coating materials are soluble in organic solvent, such as acetone or iso-propylisopropyl alcohol, whereas useful swellable agents are insoluble in organic solvents and are in a non-swollen state in these solvents. In one aspect, a coating suspension comprises Eudragit, e.g. Eudragit L100-55, as enteric coating material and sodium starch glycolate as swellable agent in a mixture of an organic solvent and a small percentage of water, for example acetone:water [97:3, v/v]. The water serves as a plasticizer. In another embodiment, the coating suspension comprises Eudragit L100-55 and Ac-Di-Sol as swellable disintegrant, in a mixture of alcohol and water, for example ethanol:water [96:4, v/v]. Suitable additives include talc and triethyl citrate.

The ingredients are mixed e.g. using a magnetic stirrer, until a homogeneous coating solution or suspension is obtained which is then applied to the outer surface of the tablet cores using methods known in the art. This may involve heating of the tablet cores, typically to a temperature of about 35-45°C. The coating composition may be applied by conventional means, for example by spraying onto the tablets. The enteric coating layer may be applied at a coating level of 1.5 to 8.0 mg/cm2, preferably 2.5 to 4.5 mg/cm2. The coating process is typically completed in 20 to 30 minutes.

The thus obtained oral dosage forms of the invention can be packaged in a container, accompanied by a package insert providing pertinent information such as, for example, dosage and administration information, contraindications, precautions, drug interactions, and adverse reactions.

### Therapeutic applications

As will be appreciated by a person skilled in the art, a dosage form according to the invention comprising an acid-labile salt is advantageously used in medicine or as a medicament. In one embodiment, it is used in combination with intravenous administration of an acid-labile salt, preferably wherein the oral dosage form is administered daily on one or more days in between intermittent intravenous boluses.

Oral dosage forms comprising thiosulphate are preferably used in a method of treatment of a subject, e.g. human or animal, that benefits from (i) an increase in serum free sulfhydryl levels; and/or (ii) *in vivo* formation of sulfide, such as hydrogen sulfide and/or sulfane sulfur or polysulfide.

In one embodiment, the invention provides the use of a controlled release thiosulphate dosage form in a method of treatment of a disease associated with hypertension, oxidative stress and/or inflammation. Also provided herein is a method of treatment of a disease associated with hypertension, oxidative stress and/or inflammation, comprising orally administering a dosage form of the invention comprising thiosulphate, preferably STS, to a subject in need thereof.

Exemplary diseases include metabolic syndrome, cardiovascular / heart disease, calciphylaxis and calcinosis cutis, inflammatory and auto-immune disease, diabetes, (chronic) kidney disease, Raynaud's disease and neurodegenerative disease.

In one specific embodiment, a dosage form of the invention comprising thiosulphate, preferably STS, is used in the (maintenance) treatment of calciphylaxis and calcinosis cutis.

Calciphylaxis, also known as calcific uremic arteriolopathy, is a devastating condition that primarily affects patients with end-stage renal disease. The lesions can progress to massive ulcerations of the subcutaneous tissue that are associated with a high degree of morbidity and mortality, usually related to sepsis. Although the pathophysiology of this condition is poorly understood, it appears to be related to a derangement in calcium-phosphate metabolism. Thus, treatments have focused on the treatment of hyperparathyroidism albeit with poor results. More recently, intravenously administered STS has emerged as a promising therapy following multiple case reports of marked disease regression following its use.

Calcinosis cutis is a condition in which calcium salts are deposited in the skin and subcutaneous tissue, for example in systemic auto-immune diseases such as systemic sclerosis. Intralesional subcutaneous injection of STS have shown promising results on resulation of lesion. However, this mode of administration is not feasible for chronic use. Systemic treatment with oral administration STS according to the present invention is advantageously used to prevent new lesion formation.

In another specific embodiment, a dosage form of the invention comprising thiosulphate, preferably STS, is used in the treatment of heart failure, including heart failure with preserved ejection fraction (HFpEF). Heart failure (HF) is a growing global health problem, with high morbidity and mortality, and the leading cause for hospitalizations in patients above 65 years of age [1]. HF can be divided into three types: HF with a preserved ejection fraction (EF >50%; HFpEF), HF with mid-range ejection fraction (EF 40-49%; HFmrEF) and HF with reduced ejection fraction (EF <40%; HFrEF) [2]. HFpEF is characterized by an increase in left ventricular (LV) wall thickness and/or left atrial size as indication of increased LV filling pressures. Additionally, impaired LV filling, also referred to as diastolic dysfunction, is observed in these patients [3]. Although diastolic dysfunction is highly prevalent in HFpEF, the disease is viewed as a complex syndrome in which cardiac and non-cardiac determinants contribute to the impairment of the cardiovascular reserve [4]. HFpEF is more predominant in elderly postmenopausal women and is closely associated with a history of hypertension as well as obesity or metabolic syndrome and CKD [5]. Potentially effective treatment options exist for HFrEF and HFmrEF, but this is not true for HFpEF [6]. This may well be because underlying mechanisms are poorly understood and are complicated by the presence of multiple interrelated comorbidities such as obesity, diabetes mellitus type 2 and hypertension. However, generalized disturbances in microvascular perfusion appear to be a unifying characteristic [7].

### LEGEND TO THE FIGURE

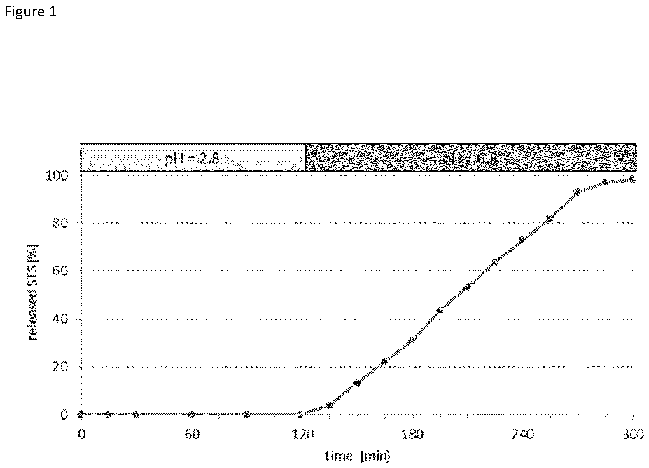

Figure 1. Dissolution curve of a representative slow release STS tablet according to example 1 coated according to example 3. The two-stage dissolution test was carried out according to example 4.

### EXPERIMENTAL SECTION

### Materials and Methods

### Materials

Sodium Thiosulfate (obtained from Boom BV, Meppel, Netherlands).
Carbopol 71G NF (from Lubrizol)
Eudragit L100-55 (from Evonik)
Magnesium Stearate (from BUFA BV)
Pharmacel ph102 (from DFE Pharma)
Primellose (from DFE Pharma)
Talc (from BUFA BV)

### Example 1: preparation of slow release STS tablet cores comprising a polymer matrix.

Tablets containing 600 mg (75 w%) of sodium thiosulfate (STS) with the composition as given below, were produced.

| **Composition:** | | |
|---|---|---|
| Sodium Thiosulfate | 75 w% | 600 mg |
| Carbopol 71G | 10 w% | 80 mg |
| Eudragit L100-55 | 14.5 w% | 116 mg |
| Magnesium Stearate | 0.5% | 4 mg |

### Process:

The STS, Carbopol and Eudragit were mixed in a Turbula mixer at 90 rpm for 10 minutes. After the addition of the magnesium stearate, mixing at 90rpm in the Turbula mixer was continued for another 2 minutes. Biconvex round tablets with a diameter of 13 mm and a weight of 800 mg were compressed with a compression force of 20 kN. The tablet crushing strength was around 178 N.

### Example 2: Preparation of immediate release STS tablet cores (comparative example)

Tablets containing 600 mg of STS with the composition as given below, were produced.

| **Composition:** | | |
|---|---|---|
| Sodium Thiosulfate | 75% | 600 mg |
| Pharmacel ph102 | 20.5% | 164 mg |
| Primellose | 4% | 32 mg |
| Magnesium Stearate | 0.5% | 4 mg |

### Process:

The STS, Pharmacel and Primellose were mixed in a Turbula mixer at 90 rpm for 10 minutes. After the addition of the magnesium stearate mixing at 90rpm in the Turbula mixer was continued for another 2 minutes. Biconvex round tablets with a diameter of 13 mm and a weight of 800 mg were compressed with a compression force of 30 kN. The tablet crushing strength was around 78N.

### Example 3: coating of tablet cores with enteric coating

Tablet cores of Examples 1 and 2 were coated in a heated mini-rotating drum (non-perforated) coater equipped with a spray nozzle. The following coating suspension was used:

**Coating composition:**

| | |
|---|---|
| Eudragit L100-55 | 3.5 gram |
| Talc | 1.5 gram |
| AC-DI-SOL | 1.5 gram |
| Triethyl citrate | 0.35 gram |
| Ethanol 96 v% | 100 ml (evaporated during coating) |

| | |
|---|---|
| Eudragit was first dissolved in ethanol, after which the talc and Ac-Di-Sol (swellable disintegrant) were suspended using a magnetic stirrer until a homogeneous suspension was obtained. | |

The tablet cores were heated to a temperature of about 40°C and the coating suspension was sprayed onto the tablets until a coating level of about 4.0 mg/cm² (which is about 16 mg). The coating process was completed in 20 to 25 minutes.

### Example 4: Dissolution testing

The coated STS tablet cores obtained in Example 3 were then evaluated for their release properties. To that end, dissolution testing was performed in a USP dissolution apparatus II (paddle apparatus) operated at a paddle speed of 50 RPM and a temperature of the dissolution medium of 37°C. The dissolution (release) of STS from the different tablets was measured using a conductivity assay (however any other suitable analytical method can be used too).

In the first stage of the dissolution test, a medium consisting of 0.0035 M citrate buffer pH 2.8 was used. This stage lasted 120 minutes. During the second stage, a 0.006 M potassium phosphate buffer pH 6.8 was used. This stage lasted 60 to 240 minutes, depending on the dissolution rate of the sodium thiosulfate.

To demonstrate the efficacy of the enteric coating and the matrix composition of the core to prevent salt release in the acid stage, the amount of STS dissolved after 120 minutes (the first acid stage) was determined. To demonstrate the slow release during the second stage, the total time (including the time taken for the acid stage) required to dissolve 50% and the time to dissolve 80% of the STS was determined.

The results obtained are summarized in table 1 below.

### Example 5: dissolution behaviour of coated slow release tablets

The tablet cores according to example 1 were coated as described in example 3. The dissolution rate of STS was analysed as described in example 4. To demonstrate the efficacy of the enteric coating and the matrix composition of the core to prevent release in the acid stage, the amount of STS dissolved after 120 minutes (the first acid stage) is given. To demonstrate the slow release during the second stage (after the 120 minutes acid) the total time (including the time taken for the acid stage) required to dissolve 50% and the time to dissolve 80% of the sodium thiosulfate are given below.

**Table 1**

| | Slow release coated STS core of the invention (Ex. 1 and 3) | Fast release coated STS core (Ex. 2 and 3) |
|---|---|---|
| Amount of drug released after 120 minutes | 0% | 0% |
| Time to release 50 w% of sodium thiosulfate | 206 minutes | 131 minutes |
| Time to release 80 w% of sodium thiosulfate | 254 minutes | 135 minutes |

A representative dissolution curve of a slow release STS tablet according to the invention is shown in Figure 1.

These data demonstrate that the tablets of Example 1 comprising both a hydrophilic matrix core combined with an enteric coating possess the desired release profile:
- no release at all in acid (first 120 minutes), which protects the thiosulfate from reacting with the hydrochloric acid in the stomach
- at pH 6.8 a slow release of the STS over a period of more than 120 minutes once the second stage of the dissolution test is reached (simulated intestinal tract), which prevents the occurrence of high salt concentrations in the small intestine.

Comparison with the release behaviour of the core of Example 2 shows that this desired slow release behaviour is obtained by the unique core containing a matrix of release controlling polymers.

### Example 6: dissolution behaviour of slow release tablet cores (not coated)

The uncoated tablet cores according to example 1 were tested. The dissolution rate of STS was analysed as described in example 4.

To demonstrate the necessity of an enteric coating the time required to release 50% of the STS and the time required to release 80% of the sodium thiosulfate was measured in the dissolution test. The results are given in table 2 below:

**Table 2**

| | |
|---|---|
| Time to release 50% of sodium thiosulfate | 33 minutes |
| Time to release 80% of sodium thiosulfate | 63 minutes |

These data show that, also for a slow release core tablet of the invention, an outer enteric coating is required to obtain the desired level of protection from gastric acid.

Furthermore, a comparison with the release behaviour (206 min/254 min) found in Example 5 indicates that the interaction of the hydrophilic core matrix with the enteric coating results in an unexpected and surprising decrease in the release rate of STS from the tablet. In case the enteric coating would merely provide a protecting layer and not affect STS release during the second (neutral) stage of the dissolution test, the time to reach 50 w% STS release was expected to be about 153 minutes (120 + 33 minutes) while 80 w% release would be reached after about 183 minutes (120 + 63 minutes). Instead, 50% release was only found after 206 minutes and that of 80% release at 254 minutes. This indicates a significant delay of 53 and 71 minutes, respectively. These findings indicate that in an oral dosage form as herein disclosed there is an unexpected synergistic effect between the core matrix composition and the coating in slowing down the release of STS.

### Example 7: Dissolution behaviour of damaged coated STS tablets

Tablet cores according to examples 1 and 2 were coated as described in example 3. After the enteric coating was applied, a scratch (deeper than the coating) of approximately 3 mm in length was made into the coating on both sides of the tablet using a razor blade. The dissolution rate of STS was analysed as described in example 4. In case of the tablets comprising the hydrophilic polymer matrix according to Example 1, only 4.8 w% of the total STS was released after 120 minutes. In contrast, tablet cores prepared according to Example 2 showed that that a significant amount (49 w%) of sodium thiosulfate was dissolved in the first acid stage.

This demonstrates that, in spite of the scratch, the release of the STS from a STS tablet core of the invention is still very minimal during the acid stage. Thus, a further advantage of the current combination of a pH sensitive hydrophilic polymer core and the pH-sensitive coating can be found in the fact that with this combination the formulation is effectively protected from degradation by acid, even when the coating is damaged.

### Example 8: Coated slow release tablets containing 300 mg sodium thiosulfate

Tablet cores containing 300 mg of sodium thiosulfate with the composition as given below, were produced.

**Composition:**

| | | |
|---|---|---|
| Sodium Thiosulfate | 70 w% | 300 mg |
| Carbopol 71G | 19.5 w% | 83 mg |
| Eudragit L100-55 | 10 w% | 43 mg |
| Magnesium Stearate | 0.5 w% | 2 mg |

### Process:

STS, Carbopol and Eudragit were mixed in a Turbula mixer at 90 rpm for 10 minutes. After the addition of the magnesium stearate, mixing at 90rpm in the Turbula mixer was continued for another 2 minutes.

Biconvex round tablets with a diameter of 9 mm and a weight of 428 mg were compressed with a compression force of 15 kN. The tablet crushing strength was around 208 N. The tablet cores were heated to a temperature of about 40°C and the coating was sprayed onto the tablets as described in Example 3 until a coating level of around 3.0 mg/cm2. The coating process was completed in around 20 minutes.

Dissolution testing was performed as described in example 4.

The results of the dissolution test are given in table 3.

**Table 3**

| | |
|---|---|
| Amount of drug released after 120 minutes | 0% |
| Time to release 50% of sodium thiosulfate | 217 minutes |
| Time to release 80% of sodium thiosulfate | 286 minutes |

This result shows that also the 300 mg oral dosage form provides the desired release profile, albeit somewhat slower than that of the 600 mg tablet, which can be explained by the different size of the tablet.

### References

1. Lam CS, Donal E, Kraigher-Krainer E, Vasan RS. Epidemiology and clinical course of heart failure with preserved ejection fraction. Eur J Heart Fail. 2011; 13(1): 18-28.
2. Borlaug BA, Paulus WJ. Heart failure with preserved ejection fraction: pathophysiology, diagnosis, and treatment. Eur Heart J. 2011;32(6):670-9.
3. Ponikowski P, Voors AA, Anker SD, et al. 2016 ESC Guidelines for the diagnosis and treatment of acute and chronic heart failure: The Task Force for the diagnosis and treatment of acute and chronic heart failure of the European Society of Cardiology (ESC) Developed with the special contribution of the Heart Failure Association (HFA) of the ESC. Eur Heart J. 2016;37(27):2129-200.
4. Lourenco AP, Leite-Moreira AF, Balligand JL, et al. An integrative translational approach to study heart failure with preserved ejection fraction: a position paper from the Working Group on Myocardial Function of the European Society of Cardiology. Eur J Heart Fail. 2018;20(2):216-27.
5. Beale AL, Meyer P, Marwick TH, et al. Sex Differences in Cardiovascular Pathophysiology: Why Women Are Overrepresented in Heart Failure With Preserved Ejection Fraction. Circulation. 2018; 138(2): 198-205.
6. Dunlay SM, Roger VL, Redfield MM. Epidemiology of heart failure with preserved ejection fraction. Nat Rev Cardiol. 2017;14(10):591-602.
6. Juilliere Y, Venner C, Filippetti L, et al. Heart failure with preserved ejection fraction: A systemic disease linked to multiple comorbidities, targeting new therapeutic options. Arch Cardiovasc Dis. 2018;111(12):766-81.
8. Candela J, Wang R, White C. Microvascular Endothelial Dysfunction in Obesity Is Driven by Macrophage-Dependent Hydrogen Sulfide Depletion. Arterioscler Thromb Vasc Biol. 2017;37:889-99.
9. Greaney JL, Kutz JL, Shank SW, et al. Impaired Hydrogen Sulfide-Mediated Vasodilation Contributes to Microvascular Endothelial Dysfunction in Hypertensive Adults. Hypertension. 2017;69(5):902-9.
10. van den Born JC, Frenay AR, Bakker SJ, et al. High urinary sulfate concentration is associated with reduced risk of renal disease progression in type 2 diabetes. Nitric Oxide. 2016;55-56:18-24.
11. Koning AM, Meijers WC, Minović I, et al. The fate of sulfate in chronic heart failure. Am J Physiol Heart Circ Physiol. 2017;312(3):H415-21.
12. van den Born JC, Frenay AS, Koning AM, et al. Urinary Excretion of Sulfur Metabolites and Risk of Cardiovascular Events and All-Cause Mortality in the General Population. Antioxid Redox Signal. 2019;30(17):1999-2010.
13. van den Berg E, Pasch A, Westendorp WH, et al. Urinary sulfur metabolites associate with a favorable cardiovascular risk profile and survival benefit in renal transplant recipients. J Am Soc Nephrol. 2014;25(6): 1303-12.
14. Koj A, Frendo J, Janik Z. [35s]thiosulphate oxidation by rat liver mitochondria in the presence of glutathione. Biochem J. 1967; 103(3):791-5.
15. Peng T, Zhuo L, Wang Y, et al. Systematic review of sodium thiosulfate in treating calciphylaxis in chronic kidney disease patients. Nephrology (Carlton). 2018;23(7):669-75.
16. Sen U, Vacek TP, Hughes WM, et al. Cardioprotective role of sodium thiosulfate on chronic heart failure by modulating endogenous H2S generation. Pharmacology. 2008;82(3):201-13.
17. Snijder PM, Frenay AR, Koning AM, et al. Sodium thiosulfate attenuates angiotensin II-induced hypertension, proteinuria and renal damage. Nitric Oxide. 2014;42:87-98.
18. Snijder PM, Frenay AR, de Boer RA, et al. Exogenous administration of thiosulfate, a donor of hydrogen sulfide, attenuates angiotensin II-induced hypertensive heart disease in rats. Br J Pharmacol. 2015; 172(6): 1494-504.
19. Nguyen ITN, Klooster A, Minnion M, et al. Sodium Thiosulfate Improves Renal Function and Oxygenation in L-NNA Induced Hypertensive Rats. Kidney Int. 2020;98(2):366-377.
20. Nguyen ITN, Wiggenhauser LM, Bulthuis M, et al. Cardiac Protection by Oral Sodium Thiosulfate in a Rat Model of L-NNA-Induced Heart Disease. Front Pharmacol. 2021; 12:650968.
21. AlBugami MM, Wilson JA, Clarke JR, et al. Oral sodium thiosulfate as maintenance therapy for calcific uremic arteriolopathy: A case series. Am J Nephrol. 2013;37(2): 104-9.
22. Asplin JR, Donahue SE, Lindeman C, et al. Thiosulfate reduces calcium phosphate nephrolithiasis. J Am Soc Nephrol. 2009;20(6): 1246-53.
23. Terstappen F, Clarke SM, Joles JA, et al. Sodium Thiosulfate in the Pregnant Dahl Salt-Sensitive Rat, a Model of Preeclampsia. Biomolecules. 2020; 10(2).
24. Senel S, Capan Y, Hincal AA. Factors affecting the formulation of sustained release potassium chloride tablets. Pharmazie, 1991; 46(11):792-5
25. Caraballo I, Millan M, Rabasco AM. Relationship between drug percolation threshold and particle size in matrix tablets. Pharm Res. 1996; 13(3):387 -90.
26. Boggiano BG, Gleeson M. Gastric acid inactivation of erythromycin stearate in solid dosage forms. J Pharm Sci, 1976;65(4):497-502
27. Altinoz MA, Ozpinar A, Ozpinar A, et al. Methenamine's journey of 160 years: Repurposal of an old urinary antiseptic for treatment and hypoxic radiosensitization of cancers and glioblastoma. Clin Exp Pharmacol Physiol. 2019;46(5):407 -12.

## Claims

1. A pharmaceutical oral dosage form for controlled delivery of an acid-labile salt, the dosage form comprising
a core comprising the acid-labile salt in a polymer matrix comprising a combination of (i) a pH-sensitive hydrophilic methacrylic acid-methyl methacrylate copolymer and (ii) a carbomer, wherein the acid-labile salt makes up at least 50wt% of the core; and wherein the outer surface of the core is provided with an enteric coating layer.

2. Dosage form according to claim 1, wherein the acid-labile salt makes up at least 60 wt%, preferably at least 65 wt% of the core.

3. Dosage form according to claim 1 or 2, wherein the acid-labile salt is selected from the group consisting of a salt of a substituted benzimidazole, a thiosulphate salt and erythromycin salt.

4. Dosage form according to claim 3, wherein the acid-labile salt comprises or consists of a thiosulphate salt, preferably sodium thiosulphate (STS).

5. Dosage form according to any one of the preceding claims, wherein the polymers in the matrix are a binary mixture of a pH-sensitive methacrylic acid-methyl methacrylate copolymer and a carbomer, preferably wherein the pH-sensitive methacrylic acid-methyl methacrylate copolymer makes up 5 to 30% of the core mass and the carbomer makes up 5 to 35% of the core mass.

6. Dosage form according to any one of the preceding claims, wherein said carbomer is a synthetic high-molecular-weight polyacrylic acid cross-linked with allyl pentaerythritol.

7. Dosage form according to any one of the preceding claims, wherein the weight ratio between (i) the pH-Sensitive methacrylic acid-methyl methacrylate copolymer and (ii) the carbomer is in the range of 1 : 0.4 to 1: 3, preferably 1 : 0.55 to 1: 2.5, more preferably 1 : 0.7 to 1 : 2.

8. Dosage form according to any one of the preceding claims, wherein the enteric coating layer is designed to release acid-labile salt at a pH above 5.0, preferably at a pH above 6.0, most preferably at a pH above 6.5.

9. Dosage form according to any one of the preceding claims, wherein the enteric coating layer comprises (a) a pH-sensitive methacrylic acid-methyl methacrylate copolymer, and optionally (b) a swellable disintegrant and (c) talc, preferably wherein the swellable disintegrant is present in the coating in a non-percolating way.

10. Dosage form according to any one of the preceding claims **characterized in that**, when subjected to a two stage *in vitro* dissolution test at 37°C, the first stage carried out for 2 hours in a media that has a pH of below 5.0 followed by a second stage carried in a media that comprises a phosphate buffer pH 6.8, the dosage form has a dissolution profile wherein up to 10%, preferably essentially none, of the acid-labile salt is released in the first stage and wherein during the second stage 35-65 w% of the dose is released after 90 minutes and more than 75 w% of the dose after 180 minutes.

11. Dosage form according to any one of the preceding claims, for use in medicine or for use as a medicament.

12. Dosage form for use according to claim 11, for use in combination with intravenous administration of an acid-labile salt, preferably wherein the oral dosage form is administered daily on one or more days in between intermittent intravenous boluses.

13. Dosage form according to any one of claim 4 and those depending thereon, for use in a method of treatment of a subject that benefits from an increase in serum free sulfhydryl levels / *in vivo* formation of sulfide, such as hydrogen sulfide and/or polysulfide.

14. Dosage form according to any one of claim 4 and those depending thereon, for use in a method of treatment of a disease associated with hypertension, oxidative stress and/or inflammation, preferably wherein the disease is selected from the group consisting of metabolic syndrome, cardiovascular / heart disease, calciphylaxis, inflammatory and auto-immune disease, diabetes, (chronic) kidney disease, Raynaud's disease and neurodegenerative disease.

15. A method for the manufacture of a dosage form according to any one of claims 1-10, comprising the steps of:
(i) providing a powder mixture comprising acid-labile salt, a pH-sensitive hydrophilic methacrylic acid-methyl methacrylate copolymer, a hydrophilic carbomer and optional pharmaceutically acceptable excipient(s), wherein the acid-labile salt makes up at least 50wt% of the mixture;
(ii) processing the mixture into a tablet core, preferably by compression or extrusion;
(iii) providing a coating composition comprising an enteric coating polymer, and optionally a swellable disintegrant, and applying the coating composition onto the tablet core.

16. A method of treatment of a disease associated with hypertension, oxidative stress and/or inflammation, preferably wherein the disease is selected from the group consisting of metabolic syndrome, cardiovascular / heart disease, calciphylaxis, inflammatory and auto-immune disease, diabetes, (chronic) kidney disease, Raynaud's disease and neurodegenerative disease, comprising orally administering a dosage form according to any one of claim 4 and those depending thereon, to a subject in need thereof.

17. Method according to claim 16, for the treatment of heart failure, preferably heart failure with preserved ejection fraction (HFpEF).
